(19) Europäisches Patentamt
European Patent Office
Office européen des brevets

(11) **EP 4 773 049 A1**

(12) **EUROPEAN PATENT APPLICATION**
published in accordance with Art. 153(4) EPC

(43) Date of publication:
**08.07.2026 Bulletin 2026/28**

(21) Application number: **24859178.6**

(22) Date of filing: **09.07.2024**

(51) International Patent Classification (IPC):
*G06N 20/00* (2019.01)   *C12M 1/00* (2006.01)
*C12P 1/00* (2006.01)   *G06N 3/09* (2023.01)

(52) Cooperative Patent Classification (CPC):
**C12M 1/00; C12P 1/00; G06N 3/09; G06N 20/00**

(86) International application number:
**PCT/JP2024/024784**

(87) International publication number:
**WO 2025/047133 (06.03.2025 Gazette 2025/10)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **31.08.2023 JP 2023140894**

(71) Applicant: **Chitose Laboratory Corp.
Kawasaki-shi, Kanagawa 216-0041 (JP)**

(72) Inventors:
  • **SAKAGUCHI Kohei
    Kawasaki-shi, Kanagawa 216-0041 (JP)**
  • **ITO Toshiaki
    Kawasaki-shi, Kanagawa 216-0041 (JP)**
  • **ONIMARU Koh
    Kawasaki-shi, Kanagawa 216-0041 (JP)**
  • **KAWAI Tetsushi
    Kawasaki-shi, Kanagawa 216-0041 (JP)**
  • **KIKUCHI Ryota
    Nagoya-shi, Aichi 467-0065 (JP)**

(74) Representative: **Plasseraud IP
104 Rue de Richelieu
CS92104
75080 Paris Cedex 02 (FR)**

(54) **CONTROL VARIABLE OPTIMIZATION METHOD, BIORESOURCE PRODUCTION METHOD, AND BIORESOURCE PRODUCTION SYSTEM**

(57)   Provided are a control variable optimization method capable of determining improved culture conditions using a predictive model based on machine learning, and a bioresource production method and a bioresource production system using the same.

A bioresource production system S according to another aspect of the present invention includes: a cultivation system B for performing bioresource production; and a control variable optimization system A for optimizing control variables obtained from the cultivation system.

The control variable optimization system separates the control variables into initial variables and manipulated variables, creates predictive models adapted to the initial variables and the manipulated variables, respectively, and optimizes the control variables by combining the predictive models.

FIG.1

## Description

[Technical Field]

The present invention relates to a control variable optimization method, a bioresource production method, and a bioresource production system.

[Background of invention]

Bioresource production systems play an important role in fermentation technology, which uses microorganisms to produce useful proteins and antibiotics; cell cultivation technology, which cultivates animal and plant cells to produce pharmaceuticals and the like; and algae cultivation technology, which cultivates microalgae and produces useful organic matter through photosynthesis using atmospheric carbon dioxide.

Known technologies related to bioresource production systems include, for example, mathematical modeling (Non-Patent Document 1 below), experimental design (Non-Patent Document 2 below), and dimensionality reduction (Non-Patent Document 3 below).

The mathematical model shown in Non-Patent Document 1 below constructs a dynamic mathematical model consisting of antibody production amount, viable cell count, apoptotic cell count, dead cell count, culture volume, and pH. The parameters of the mathematical model are estimated based on results of culture tests conducted at different pH values, and ultimately an optimal control value of pH is predicted using the mathematical model with the estimated parameters.

Furthermore, the experimental design method shown in Non-Patent Document 2 below discloses a technique in which several medium components and fermentation parameters are optimized using a greedy method or the like, important factors are screened, and then further optimization is performed using an experimental design or the like.

In addition, in the dimension reduction shown in Non-Patent Document 3 below, a dimension reduction method that combines Boruta, one of the dimension reduction methods, with variable importance (VIP) in PLS is applied to near-infrared spectral data, and lactate and glucose concentrations are predicted from the dimension-reduced near-infrared spectral data using a machine learning model.

[Prior art documents]

[Non-Patent Documents]

[Non-Patent Document 1]
Optimization of a pH-shift control strategy for producing monoclonal antibodies in Chinese hamster ovary cell cultures using a pH-dependent dynamic model, T. Hogiri et al., J. Biosci. Bioeng., 2018, 125(2).
[Non-Patent Document 2]
Optimization of heterologous production of Bacillus ligniniphilus L1 laccase in Escherichia coli through statistical design of experiments, I. Mutanda et al., Microbiol. Res., 2023, 274.
[Non-Patent Document 3]
Transfer learning and wavelength selection method in NIR spectroscopy to predict glucose and lactate concentrations in culture media using VIP-Boruta, H. Kaneko et al., Anal. Sci. Adv., 2021, 2, 470-479.

[Summary of invention]

[Problems to be solved by invention]

However, since mathematical models cannot fully represent the complexities of real cultivation systems, there is a problem in that it is difficult to predict cultivation systems with high accuracy using mathematical models. Furthermore, there is a problem in that when only a single machine learning model is used, it is difficult to optimize control variables having different characteristics.

Furthermore, greedy algorithm-based optimization methods are effective when each control variable is independent and there is no interaction among the control variables; however, since control variables in cultivation often interact with each other, there is a problem in that greedy algorithm-based optimization methods are likely to obtain only local solutions.

Furthermore, experimental design allows optimization even when there are interactions; however, when there are many types of control variables or when attempting to consider time-varying changes in each control variable, the number of combinations becomes enormous, and even experimental design cannot fully optimize. Optimization requires a huge number of experiments, but as a characteristic of bioresource production, there is a limit to the number of cultures that can be performed at one time, and each culture may be expensive and time-consuming. Furthermore, when the number of control variables increases, humans cannot fully understand and interpret the experimental results.

For the reasons described above, experimental design cannot fully address optimization problems involving many control variables, and therefore, at present, the number of control variables is often limited, or cultivation is conducted under constant control conditions.

In view of the above problems, the present invention aims to provide a control variable optimization method capable of determining improved culture conditions using a predictive model based on machine learning, as well as a bioresource production method and a bioresource production system using the control variable optimization method.

[Means for Solving the Problem]

A control variable optimization method according to one aspect of the present invention that solves the above problem is characterized in that the control variable is separated into an initial variable and a manipulated variable, predictive models adapted respectively to the initial variable and the manipulated variable are created, and the control variable is optimized by combining the predictive models.

Furthermore, in this aspect, although the present invention is not limited thereto, it is preferable to calculate priorities of the initial variable and the manipulated variable from a gradient of the objective variable with respect to the control variable, and to determine the order in which the initial variable and the manipulated variable are optimized based on the calculated priorities.

Furthermore, in this aspect, although the present invention is not limited thereto, it is preferable to optimize the control variable by repeatedly using two models adapted respectively to the initial variable and the manipulated variable and recursively performing inverse analysis until optimal control values of the initial variable and the manipulated variable converge.

Furthermore, in this aspect, although the present invention is not limited thereto, it is preferable to optimize the control variable by assigning medium temperature and pH to the manipulated variables, assigning medium components and inoculation amount to the initial variables, and combining two models adapted respectively to the initial variables and the manipulated variables.

Furthermore, in this aspect, although the present invention is not limited thereto, it is preferable to perform dimension reduction by extracting coefficients that minimize approximation error through projection of a large number of explanatory variables used in each model onto a Gompertz curve.

Furthermore, in this aspect, although the present invention is not limited thereto, it is preferable to present, when optimal control values of the initial variable and the manipulated variable are calculated, a distribution of predicted values of the control variable and a relative position of the optimal control value with respect to training data.

Furthermore, in this aspect, although the present invention is not limited thereto, it is preferable to separate the manipulated variables into time-indexed variables so that they can be treated in the same manner as the initial variables.

A bioresource production method according to another aspect of the present invention comprises performing cultivation using control variables optimized by a control variable optimization method in which the control variables are separated into initial variables and manipulated variables, predictive models adapted respectively to the initial variables and the manipulated variables are created, and the control variables are optimized by combining the predictive models.

In addition, a bioresource production system according to another aspect of the present invention comprises a cultivation system that performs bioresource production and a control variable optimization system that optimizes control variables obtained from the cultivation system, wherein the control variable optimization system separates the control variables into initial variables and manipulated variables, creates predictive models adapted respectively to the initial variables and the manipulated variables, and optimizes the control variables by combining the predictive models.

[Effect of invention]

According to the present invention, there are provided a control variable optimization method capable of determining improved culture conditions using a predictive model based on machine learning, as well as a bioresource production method and a bioresource production system using the same.

[Brief description of drawings]

[FIG. 1] Functional block diagram of an entire bioresource production system according to an embodiment.

[FIG. 2] Diagram illustrating a data processing flow of a bioresource production method according to the embodiment.

[FIG. 3] Diagram illustrating determination of priorities of control variables in the bioresource production method according to the embodiment.

[FIG. 4] Diagram illustrating dimension reduction in the bioresource production method according to the embodiment.

[FIG. 5] Diagram illustrating determination of validity of an optimal value in the bioresource production method according to the embodiment.

[FIG. 6] Diagram illustrating an overview of results of actual cultivation in the bioresource production method according to the embodiment.

[FIG. 7] Diagram illustrating an overview of application of a Gompertz curve to actual cultivation in the bioresource production method according to the embodiment.

[FIG. 8] Flowchart illustrating calculation of an optimal control value in the bioresource production method according to the embodiment.

[FIG. 9] Functional block diagram during real-time control of the entire bioresource production system according to the embodiment.

[FIG. 10] Flowchart illustrating calculation of an optimal control value during real-time control of the bioresource production system according to the embodiment.

[Detailed Description]

Hereinafter, embodiments of the present invention will be described in detail with reference to the drawings. However, the present invention can be embodied in many different forms and is not limited to the specific descriptions given in the following embodiments and examples.

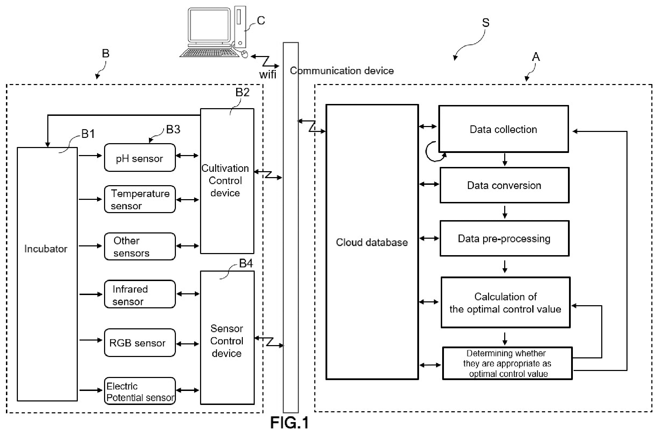

FIG. 1 is a functional block diagram of an entire bioresource production system S according to the present embodiment (hereinafter referred to as "the system").

As shown in FIG. 1, the system S comprises a cultivation system B and a control variable optimization system A that adjusts control variables for the cultivation system B. As shown in FIG. 1, the system S produces bioresources by cultivation using the cultivation system B, while control variables used in the cultivation system B are adjusted by the control variable optimization system A.

As described above, the cultivation system B of the system S is a system capable of cultivating bioresources. Specifically, the cultivation system B includes at least an incubator B1, a cultivation control device B2 that controls the incubator B1, various sensors B3, and a sensor control device B4 that controls the sensors B3.

The incubator B1 of the system S is an apparatus including a culture vessel for culturing bioresources, and by introducing a target bioresource into the incubator B1, the bioresource can be cultivated and proliferated (i.e., produced).

The specific structure of the incubator B1 is not limited as long as it has the above functions, but may include, for example, a culture vessel such as a jar fermenter, a temperature control device configured to control temperature of the jar fermenter, a rotating device configured to rotate the culture vessel, an acid or base supply device configured to adjust pH, an oxygen supply device configured to supply oxygen, a carbon dioxide supply device configured to supply carbon dioxide, and various other devices, and may further include an observation device configured to observe an interior of the culture vessel; however, the structure is not limited thereto.

In addition, not only a bioresource to be proliferated is introduced into the culture vessel of the incubator B1 of the system S, but also a culture medium or culture fluid may be added as necessary.

Furthermore, in the system S, the term "bioresources" refers to resources derived from living organisms, and more specifically includes, but is not limited to, plants, microorganisms, cells, algae, bacteria, viruses themselves, and useful products produced by activities thereof.

Specifically, the system S can be applied to microbial manufacturing, pharmaceutical production using cells, algal manufacturing, and agriculture. For example, in a microbial manufacturing process in which substances are produced by microorganisms, initial variables include medium components, inoculation amount, metabolic switching time, feed start time, culture medium volume at the start, autoclave time and temperature, feed solution, feed solution concentration, and pH adjustment solution, and manipulated variables include temperature (Temp), pH, agitation speed (Agit), dissolved oxygen (DO), aeration volume, feed rate, temperature dead zone, pressure, and oxygen concentration.

Therefore, the microbial manufacturing process can be controlled using the initial variables and the manipulated variables.

Also, in pharmaceutical production using cells, for example, in antibody (protein) production using CHO cells (animal cells), initial variables include medium components, inoculation amount, metabolic switching time, feed start time, culture medium volume at the start, autoclave time and temperature, feed solution, feed solution concentration, and pH adjustment solution, and manipulated variables include temperature (Temp), pH, agitation speed (Agit), dissolved oxygen (DO), aeration volume, feed rate, temperature dead zone, pressure, and oxygen concentration.

Therefore, the pharmaceutical production process using cells can be controlled using the initial variables and the manipulated variables.

Furthermore, in algal manufacturing, in a process in which substances are produced using algae, initial variables include medium components, inoculation amount, metabolic switching time, feed start time, culture medium volume at the start, autoclave time and temperature, feed solution, feed solution concentration, and pH adjustment solution, and manipulated variables include temperature (Temp), pH, agitation speed (Agit), dissolved oxygen (DO), aeration volume, feed rate, temperature dead zone, pressure, oxygen concentration, $CO_2$ supply rate, and light intensity.

Therefore, the algal manufacturing process can be controlled using the initial variables and the manipulated variables.

Furthermore, in agriculture, for example, in organic farming and natural farming, initial variables include chemical parameters (pH, electrical conductivity (EC), nitrogen ion concentration, phosphate ion concentration, potassium ion concentration), physical parameters (water content and maximum water holding capacity), and biological parameters (total bacterial count, total carbon content, total nitrogen content, total phosphorus content, total potassium content, C/N ratio, and C/P ratio), and manipulated variables include annual carbon input and annual nitrogen input from organic materials.

Therefore, agriculture based on organic farming and natural farming can be controlled using the initial variables and the manipulated variables.

In the system S, various sensors B3 are connected to the culture vessel.

Here, the term "sensor" refers to a device used to monitor conditions inside and around the culture vessel, and examples thereof include, but are not limited to, culture condition detection sensors that detect culture conditions, such as a pH sensor that detects pH, a temperature sensor that detects temperature, a dissolved oxygen sensor that detects dissolved oxygen concentration (DO), and an oxygen sensor that detects oxygen concentration, as well as cultivation status detection sensors that detect cultivation status, such as a carbon dioxide sensor that detects carbon dioxide concentration, an infrared sensor that detects infrared radiation, an RGB sensor that detects color, and a potential sensor that detects electric potential.

By using these various sensors, it is possible to detect the culture environment and determine the production status of bioresources. After such detection, the culture conditions in bioresource production can be adjusted by controlling the cultivation control device B2.

Furthermore, at least some of the various sensors B3 of the system S are connected to the cultivation control device B2.

Specifically, the cultivation control device B2 is connected to culture condition detection sensors, such as a pH sensor, a temperature sensor, a dissolved oxygen sensor that detects dissolved oxygen concentration (DO), and an oxygen sensor that detects oxygen concentration. The cultivation control device B2 is capable of adjusting culture conditions, such as pH, temperature, dissolved oxygen concentration, and oxygen concentration, based on detection results of these sensors.

This allows the system S to achieve and maintain optimal culture conditions.

As will be described later, data measured by at least some of the various sensors B3 is collected by the cultivation control device B2, and additional sensor data (such as jar number, version number, and serial number) is added before the data is transmitted to the optimal control variable system A.

At least some of the various sensors B3 of the system S are connected to the sensor control device B4. Specifically, the sensor control device B4 is connected to cultivation status detection sensors that detect cultivation status, such as a carbon dioxide sensor that detects carbon dioxide concentration, an infrared sensor that detects infrared radiation, an RGB sensor that detects color, and a potential sensor that detects electric potential.

By being connected to the above cultivation status detection sensors, the sensor control device B4 is capable of performing necessary control over these sensors and transmitting acquired data to devices outside the culture vessel.

As a result, not only can the cultivation status be confirmed, but the data can also be used as training data for machine learning described later.

As will be described later, data measured by at least some of the various sensors B3 is collected by the sensor control device B4, and additional sensor data (such as jar number, version number, and serial number) is added before being transmitted to the optimal control variable system A.

The system S further includes an optimal control variable system A connected to the cultivation system B. The optimal control variable system A is capable of optimizing control variables based on outputs received from the culture condition detection sensors and the cultivation status detection sensors. Details thereof will be described later.

In the system S, the cultivation system B and the optimal control variable system A are connected so as to enable transmission and reception of data. The cultivation system B and the optimal control variable system A may be connected directly by wiring, such as a cable, or may be connected via a LAN (Local Area Network), a WAN (Wide Area Network), or the like.

In the example shown in the figure, the cultivation control device B2 and the sensor control device B4 are connected to a LAN via Wi-Fi or the like, and are further connected via a WAN to the optimal control variable system A.

A specific example of a device used for connecting a LAN, a WAN, or the like to various devices is a communication device such as a modem.

Furthermore, the system S may further include a control computer C. By providing the control computer C and connecting it to the cultivation system B and the optimal control variable system A, it becomes possible to enable integrated control of these systems.

In the system S, the control computer C may be embodied as a computer per se, and the optimal control variable system A may be implemented using a general-purpose computer.

A computer is a device capable of performing predetermined processing based on input data and outputting data, and is preferably configured to include, for example, a central processing unit (CPU), a non-volatile recording medium such as a hard disk or a flash memory, a volatile recording medium such as a memory, an input device such as a keyboard or a mouse, a communication device such as a modem, and a bus connecting these components.

The computer is also preferably provided with a display device for displaying results of data processing.

Furthermore, as described above, the optimal control variable system A of the system S is capable of optimizing control variables based on outputs from the culture condition detection sensors and the cultivation status detection sensors, and this can be achieved by executing a computer program.

That is, according to the system S, a control variable optimization method can be realized by causing a computer to execute a program for optimizing control variables (a control variable optimization program; hereinafter referred to as "the present program"), and the system S can realize an optimized bioresource production method by using the optimized control variables.

This diagram shows functional blocks for explaining a flow (processing flow) of control variable optimization realized by the present program.

In other words, the optimal control variable system A in the system S functions as the respective functional blocks by executing the present program.

First, as is clear from the above description, values acquired by the various sensors B3 in the cultivation system B are uploaded and accumulated as various data in a cloud database (cloud DB) of the optimal control variable system A via a LAN, a WAN, or the like.

The cloud database can be realized by recording data in a designated area of a non-volatile recording medium, such as a hard disk of a computer.

Furthermore, among data uploaded to the cloud database, data to be processed is collected (data collection), converted into a standardized format, and prepared for subsequent processing. The converted data is then written back to the cloud database.

Furthermore, it is preferable that data acquired by the various sensors B3 be subjected not only to data conversion for unifying formats but also to preprocessing for efficiently performing specific processing.

For example, since data acquired by the various sensors B3 contains noise, it is preferable, from the viewpoint of further improving optimization accuracy, to smooth the data by performing, for example, a moving average process to remove the noise.

It is also preferable to convert measured values from each sensor into different numerical values; for example, converting RGB sensor data into Lab values or converting near-infrared sensor data into absorbance.

Data subjected to such preprocessing is then written back to the database.

Then, in the optimal control variable system A of the system S, machine learning is performed using data that has undergone the above preprocessing, a predictive model is constructed, and a control variable optimization process for calculating an optimal control value is performed.

As will be described later, data obtained by the various sensors B3 constitutes a part of the control variables, specifically manipulated variables, and the control variables are optimized together with other variables (initial variables).

Furthermore, data (control variables) calculated by the control variable optimization process is subjected to a validity determination process for determining whether the data is appropriate as an optimal value.

The validity is determined based on a relative position with respect to training data and whether the optimal control value falls within a high-value region of the objective variable distribution.

If it is determined that the data is valid, the data (control variable) is written back to the cloud database. If it is determined that the data is invalid, the process returns to the data collection step or the control variable optimization step, and the processing is performed again.

Data (control variables) that are determined to be valid in the above process are transmitted to the cultivation system B, more specifically to the cultivation control device B2. The cultivation control device B2 adjusts the culture conditions of the incubator B1 so as to correspond to the control variables, thereby optimizing production of the bioresources.

Hereinafter, details of a control variable optimization method (hereinafter referred to as "the present method") specifically performed by the optimal control variable system A will now be described in detail.

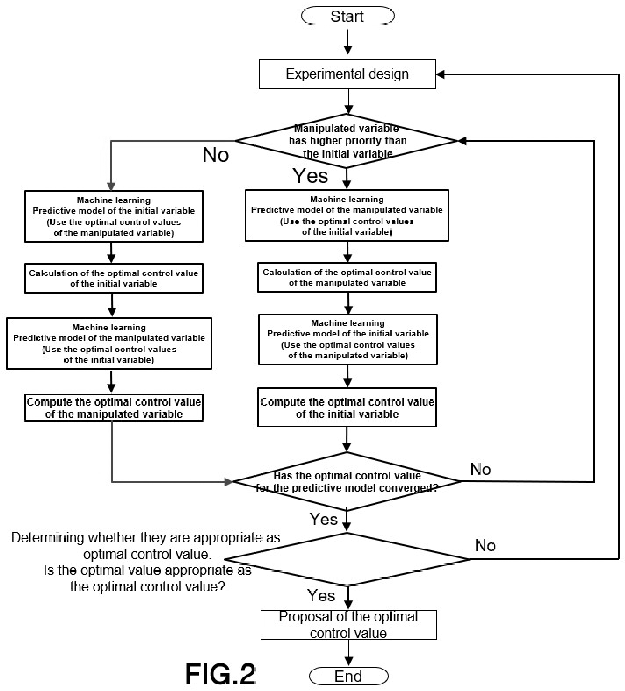

The present method is characterized in that control variables are separated into initial variables and manipulated variables, predictive models adapted respectively to the initial variables and the manipulated variables are created, and the control variables are optimized by combining these predictive models. FIG. 2 shows a data processing flow of the present method.

As shown in FIG. 2, in the present method, it is preferable to first use data obtained by experimental design. Specifically, control variables that have been varied in various ways by the present system S, and data obtained as a result of varying these control variables, are used.

As described above, in the present method, control variables are separated into initial variables and manipulated variables.

Here, a "control variable" refers to a condition (variable) required for optimizing cultivation, a "manipulated variable" refers to an observable variable that changes from moment to moment and can be controlled, and an "initial variable" refers to an observable variable that is set only once as an initial value for cultivation and does not change thereafter.

In the present method, optimization processing is performed with respect to both the initial variables and the manipulated variables.

Specific examples of the manipulated variables include, but are not limited to, medium temperature, pH, agitation speed (Agit), dissolved oxygen concentration (DO), aeration volume, feed rate, temperature dead zone, pressure, and oxygen concentration.

Specific examples of the initial variables include, but are not limited to, medium components, inoculation amount, metabolic switching time, feed start time, culture medium volume at the start, autoclave time and temperature, feed solution, feed solution concentration, and pH adjustment solution.

Furthermore, as described above, control variables are separated into manipulated variables and initial variables; however, the explanatory variables of a predictive model include not only control variables but also state variables, and are separated into manipulated variables, initial variables, and state variables.

However, state variables are observable but cannot be controlled during cultivation and are therefore not subject to optimization in the predictive model.

Accordingly, the optimization process described below is performed only on the manipulated variables and the initial variables among the explanatory variables.

Specific examples of the state variables include, for example, exhaust $CO_2$ concentration, exhaust $O_2$ concentration, RGB values, electric potential, visible light, near-infrared radiation, fluorescence, ultraviolet light (UV), viscosity, vibration, odor, respiration quotient, ventilation volume, operating time of a heating/cooling pump, operating time of a pH adjustment pump, feed weight, kLA, glucose concentration, and enzyme sensor output; however, the state variables are not limited thereto.

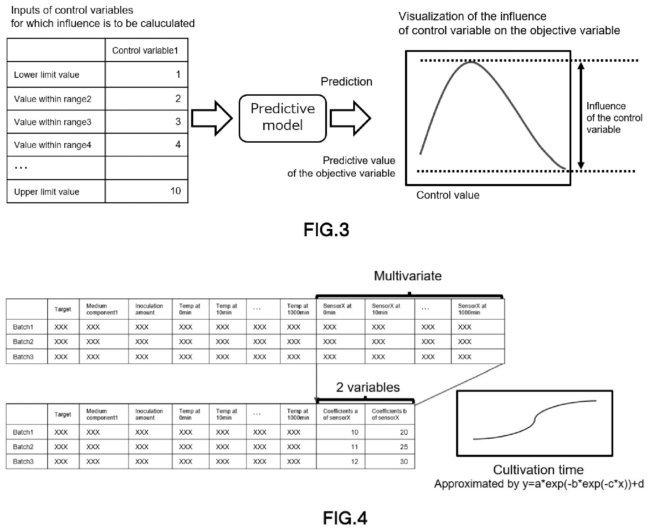

After separating the control variables into manipulated variables and initial variables, the present method determines the priority between the manipulated variables and the initial variables. FIG. 3 illustrates an example for determining such priority.

Specifically, this figure shows a method for calculating the influence of each control variable on an objective variable. Here, an "objective variable" refers to a variable serving as an index for obtaining an optimal value. When the predicted value of the objective variable exhibits a large variation, the corresponding control variable is determined to have a high priority, whereas when the variation is small, the priority is determined to be low.

As shown in the figure, candidate values that each control variable can take are generated within a predetermined setting range of the control variable, and these data are input into a machine learning model to calculate how the objective variable varies depending on the value of the control variable.

By calculating a difference between a maximum value and a minimum value of the calculated variation (i.e., an influence on the objective variable, corresponding to a gradient of the control variable with respect to the objective variable), it is possible to estimate which control variable has a greater influence.

When performing sequential optimization, it is preferable to start optimization from a control variable having a greater influence. Therefore, in the present invention, a control variable having a larger calculated influence is determined to have a higher priority and is optimized first.

After a priority among the control variables is determined by the above process, a predetermined process is performed on a manipulated variable or an initial variable that is determined to have a higher priority. The same process is performed on both the manipulated variable and the initial variable.

First, when the initial variable is determined to have a higher priority, the optimal value of the initial variable is determined using a predictive model for the initial variable. Thereafter, the optimal value of the manipulated variable is determined using a predictive model for the manipulated variable.

Conversely, when the manipulated variable is determined to have a higher priority, the optimal value of the manipulated variable is determined using the predictive model for the manipulated variable, and subsequently the optimal value of the initial variable is determined using the predictive model for the initial variable.

In other words, a characteristic feature of the present method resides in that a predictive model for the initial variable and a predictive model for the manipulated variable are constructed, and the control variables are optimized by combining these predictive models.

Note that the predictive model used for machine learning based on the manipulated variables employs training data having a data format including manipulated variables, initial variables, state variables, and objective variables, wherein the values of the manipulated variables are varied, the values of the initial variables and the state variables are fixed, and the objective variable represents a resulting value corresponding thereto.

Machine learning is performed using the training data structured in the above data format.

After completion of the machine learning, a control value of the manipulated variable that maximizes the objective variable is determined through inverse analysis of the predictive model.

Similarly, the predictive model used for machine learning based on the initial variables is similar to that described above.

Specifically, it employs training data having a data format consisting of manipulated variables, initial variables, state variables, and objective variables, wherein values of the initial variables are varied, values of the manipulated variables and the state variables are fixed, and the objective variable represents a resulting value corresponding thereto.

Machine learning is performed using the training data structured in the above data format.

After completion of the machine learning, a control value of the initial variable that maximizes the objective variable is determined through inverse analysis of the predictive model.

By the way, in the present method, the training data for the predictive model for the initial variables includes time-series state variables whose dimensionality corresponds to (the number of manipulated variables + the number of sensors) multiplied by the cultivation time (for example, when the cultivation time is 1,000 minutes, each state variable includes 1,000 time-indexed values). Therefore, if a predictive model is created without performing dimensionality reduction, prediction accuracy of the predictive model is expected to deteriorate due to multicollinearity and the curse of dimensionality. Accordingly, in the present method, it is useful to perform dimensionality reduction by approximating time-series sensor data with a Gompertz curve expressed as:

$$y = a \times \exp(-b \times \exp(-c \times x)) + d.$$

In the above example, by approximating a state variable X with the Gompertz curve, the original 1,000 time-indexed variables can be reduced to four variables, namely coefficients a, b, c, and d that determine the Gompertz curve. It is preferable to selectively use an appropriate approximating function, such as a logarithmic function, an exponential function, or the Gompertz curve, in view of biological knowledge. An illustrative example of this case is shown in FIG. 4.

In other words, in this process, dimensionality reduction is performed by projecting the enormous number of explanatory variables used in each model onto an approximating function such as the Gompertz curve and extracting coefficients that minimize the approximation error.

Although, in the explanation of the present method so far, the manipulated variables and the initial variables have been treated as different variables, in some cases the manipulated variables may be treated as initial variables.

Specifically, the manipulated variables can be divided into time-indexed variables corresponding to each cultivation time point, such as a manipulated variable X0 at 0 min, a manipulated variable X10 at 10 min, a manipulated variable X20 at 20 min, ..., and a manipulated variable Xn at n min; therefore, these variables can be treated as initial variables.

In such a case, it is possible to omit (i) the determination of priorities among the control variables and (ii) machine learning with respect to the manipulated variables. In other words, in order to enable all manipulated variables to be treated equivalently to the initial variables, it is also preferable to separate the manipulated variables into time-indexed variables.

Once the machine learning has been completed, a determination is made as to whether the optimal value obtained from the predictive model has converged. If convergence has occurred, the process proceeds to the next step. If convergence has not occurred, the process returns to the priority determination step, machine learning is performed again, and the optimal value is recalculated.

Various determination methods may be adopted to determine whether convergence has occurred. For example, it is preferable to determine whether a difference between successively calculated solutions gradually decreases and becomes smaller than a predetermined threshold.

In other words, by repeatedly using two models adapted respectively to the initial variable and the manipulated variable and recursively performing inverse analysis until the optimal control values of the initial variable and the manipulated variable converge, the control variable is optimized, whereby more accurate results are obtained.

When the optimal value has converged, a determination is made as to whether the optimal value is appropriate as the optimal control value. If it is not appropriate, the process returns to the experimental design step, and the amount of collected data is increased.

The process is continued until the control value is finally extracted in a manner that prevents the optimal value from becoming a biased or spurious solution due to the amount and bias of the training data.

On the other hand, if the optimal value is determined to be appropriate as the optimal control value, the obtained result is confirmed as the optimal control value and is presented, and the process is terminated.

However, the present method involves time-series control of manipulated variables and multivariate optimization of the initial variables, resulting in complex and multidimensional optimal conditions.

Therefore, it is difficult for humans to determine whether the proposed control values are likely to lead to good culture results.

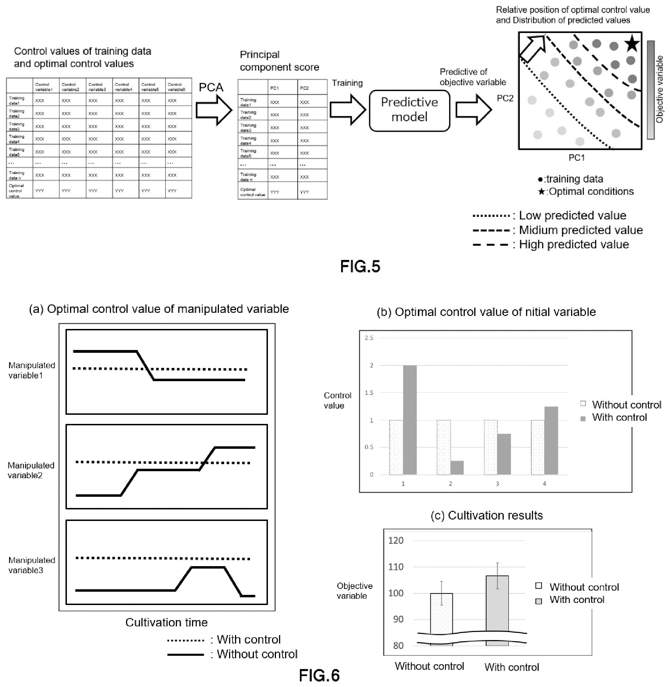

Accordingly, by reducing the dimensions of the multidimensional control variables of the training data and the optimal control value and projecting the training data and the optimal control value into the reduced-dimensional space, it becomes possible to confirm the relative position of the optimal control value and to quantitatively visualize the influence of the control variables on the objective variable. This is illustrated in FIG. 5.

In this case, a predictive model is created using the training data in the projected space as input, contours of the predicted values are calculated, and the contours are added to the above diagram to visualize the distribution of the predicted values.

This makes it possible to confirm whether the optimal control value is located within a high-value region of the objective variable distribution.

The validity of the obtained optimal control value can be determined by confirming its relative position and whether it falls within the high-value region of the objective variable distribution.

That is, when the optimal control values of the initial variables and the manipulated variables are calculated, it is preferable to present the distribution of the predicted values over the control-variable space and the relative position of the optimal control value with respect to the training data.

As described above, this embodiment can provide a control variable optimization method that can determine more optimal culture conditions using a predictive model that uses machine learning, as well as a bioresource production method and a bioresource production system that use this control variable optimization method.

To describe the effects of the present invention in more detail, in the present embodiment, optimal culture conditions are proposed by a predictive model based on machine learning, thereby enabling an improvement in microbial productivity of nearly 10% compared to culture conditions derived from conventional optimization methods.

The results of applying this technology to the actual cultivation of a certain microorganism are shown in FIG 6. This result was obtained by creating a predictive model for the cultivation of a certain microorganism, calculating the optimal conditions for a certain objective variable, and culturing the microorganism according to that model.

In addition, there are a total of seven control variables in the main culture. Conventionally, the aim is to maximize the objective variable by selecting the value of each variable, but in the present method, of the seven control variables, four are assigned as initial variables, and three are assigned as manipulated variables.

The differences between the conventional method and the present method with respect to the manipulated variables and the initial variables are shown in (a) and (b), respectively. As can be seen, the manipulated variables in the present method change over the culture time, whereas in the conventional method they must be kept constant.

Furthermore, in the present method, optimal values for the initial variables can be extracted while taking into account changes in the manipulated variables, and it can be seen that values significantly different from those selected by the conventional method are selected.

Finally, the difference in productivity is shown in FIG 6 (c). Compared to the conventional method (left bar graph), the objective variable value is higher when using the present method (right bar graph), and it can be seen that productivity is nearly 10% higher.

Furthermore, in actual cultivation, culture conditions constantly fluctuate due to factors such as weather, inoculation, and the state of the culture medium.

However, in the present method, the manipulated variables are controlled in response to such fluctuations, thereby enabling cultivation that maintains the improved productivity.

Furthermore, because culture conditions have traditionally been determined based on the experience of skilled technicians, it has been difficult to visualize the impact of control variables on the objective variable.

In contrast, in the present method, as shown in FIG. 5, dimensional reduction is performed, thereby making it possible to plot a graph in which the predicted value of the objective variable is represented on the vertical axis and the control variables are represented on the horizontal axis.

This has the advantage of visualizing the effect of the control variables on the objective variable, and the resulting information can be utilized when designing or constructing cultivation equipment.

Furthermore, as shown in FIG. 4 above, projection onto the Gompertz curve enables dimensionality reduction and improves prediction accuracy by about 35% compared to when the Gompertz curve is not used.

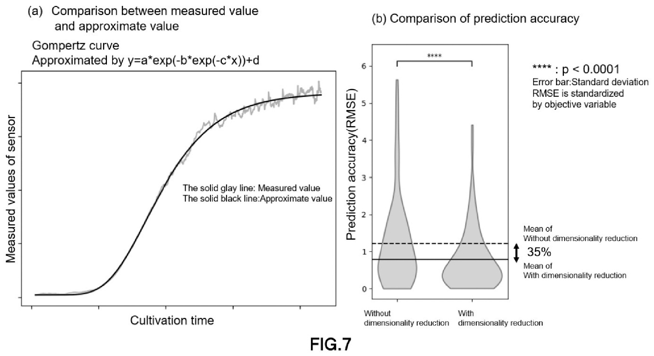

FIG. 7 shows the results of applying the present method to a microbial culture.

For a given objective variable in a microbial culture, predictive models were constructed for two cases: (i) without dimensionality reduction (three culture condition variables plus 852 time-series data points at a single wavelength measured by a visible light sensor), and (ii) with dimensionality reduction using Gompertz curve approximation (a) (three culture condition variables plus four Gompertz-curve coefficients).

The prediction accuracies (RMSE normalized with respect to the objective variable) were compared, and a paired t-test was performed to evaluate whether the difference was statistically significant. The results are shown in Figure (b) (violin plot).

In the figure, the width of the light-gray area represents the data density, the dashed line indicates the mean RMSE without dimensionality reduction, and the solid line indicates the mean RMSE with dimensionality reduction. "****" denotes $p < 0.0001$ in the corresponding paired t-test.

As shown in this figure, when dimensionality reduction was performed, the prediction accuracy was significantly improved, with the RMSE being significantly lower. On average, the improvement was approximately 35%, calculated as (RMSE without dimensionality reduction - RMSE with dimensionality reduction) / RMSE with dimensionality reduction.

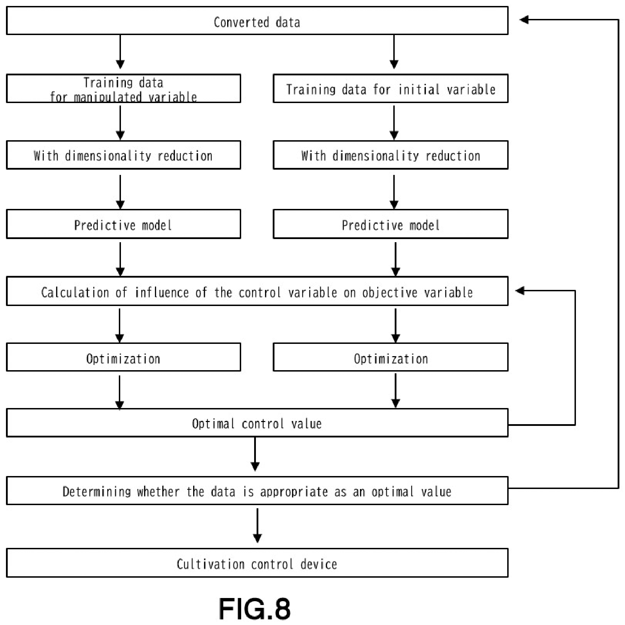

An example of a flowchart for calculating an optimal control value is shown in FIG. 8.

In this calculation, training data for the manipulated variables and training data for the initial variables are created based on the converted data. In the training data for the manipulated variables, the manipulated variables are treated as control variables, and the initial variables and the sensor data are treated as state variables.

On the other hand, in the training data for the initial variables, the initial variables are treated as control variables, and the manipulated variables and the sensor data are treated as state variables.

Here, each training dataset is converted into a dimension-reduced dataset by performing dimensionality reduction using PCA or approximation using a Gompertz curve, and by performing feature selection using correlation coefficients, clustering, regularization, or the like.

Next, the dimension-reduced dataset is used as training data to construct a predictive model using machine learning techniques such as neural networks and random forests.

Subsequently, control variables having a large impact on the cultivation are identified based on cultivation domain knowledge or by calculating the gradient of the objective variable with respect to the control variables using the model, and the priority for optimization of the initial variables and the manipulated variables is determined.

Hereinafter, a case in which the manipulated variables have a large impact will be described as an example.

In the optimization process, the optimal control value of the manipulated variable having the highest priority is first calculated using the predictive model constructed in the first step (= inverse analysis).

The data used for the inverse analysis is one of the acquired datasets that was not used for training and is used as batch data for simulation.

Next, the optimal control value of the initial variable is calculated by reflecting, in the predictive model in which the initial variable serves as the control variable, the optimal control value of the manipulated variable that is treated as a state variable in that model. Furthermore, the optimal control value of the initial variable thus calculated is reflected in the predictive model in which the manipulated variable serves as the control variable, and the optimal control value of the manipulated variable is calculated again. Thereafter, the optimization of the manipulated variable and the initial variable is performed recursively until the optimal control values converge, and finally, the converged optimal control values of the manipulated variable and the initial variable are obtained.

The multidimensional control variables of the training data and the optimal control values are subjected to dimensionality reduction using PCA or other methods, and the relative position of the optimal control values is confirmed by projecting the training data and the optimal control values onto the dimensionally reduced space.

Furthermore, a predictive model is constructed using the training data in the projected space as input, contour lines of the predicted values are calculated, and by adding these contour lines to the aforementioned figure, the distribution of the predicted values is obtained.

Thus, a figure depicting both the relative position of the optimal control values and the distribution of the predicted values is generated to determine whether the optimal control values are valid.

Then, by an operator manually inputting the optimal control value judged to be valid into the cultivation system, specifically the cultivation apparatus, in advance before starting cultivation, cultivation can be carried out in accordance with the optimal control value predicted by the predictive model based on machine learning.

Incidentally, the present method may also be applied to calculate the optimal control value while performing cultivation and controlling the cultivation in real time.

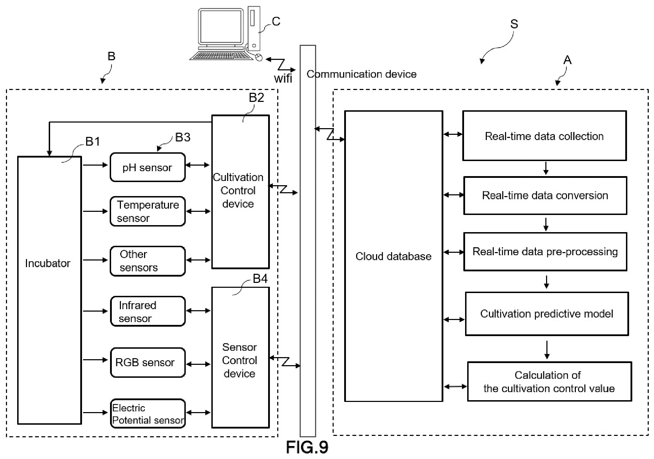

FIG. 9 shows a functional block for calculating the optimal control value during real-time control. In this block diagram, only the control variable optimization system A differs, and the other configuration is the same as that shown in FIG. 1.

In this example, the optimal control value of the manipulated variable is calculated by inputting the real-time converted data into the constructed predictive model and performing inverse analysis.

The calculated optimal control value of the manipulated variable is fed back to the cultivation control device B2 in real time, thereby enabling real-time cultivation control based on the predictive model.

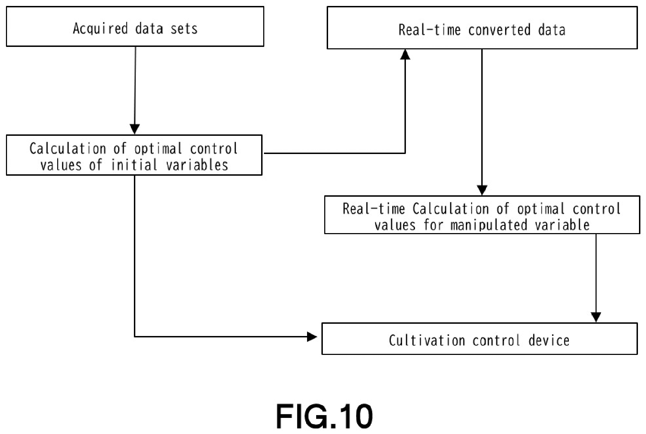

FIG. 10 shows a flowchart for calculating the optimal control value during real-time control. In this case, previously acquired data is used.

Specifically, before the start of cultivation, one of the previously acquired datasets that was not used for training is used as data for inverse analysis to calculate the optimal control value of the initial variable.

After this calculation, the optimal control value of the initial variable is input in advance to the cultivation control device B2.

In addition, the calculated optimal control value of the initial variable is reflected as a state variable in the real-time converted data.

After the start of cultivation, the optimal control value of the manipulated variable is calculated in real time using the real-time converted data as input.

The calculated optimal control value of the manipulated variable is fed back to the cultivation control device B2 in real time, thereby realizing real-time cultivation control based on the predictive model.

[Industrial Applicability]

The present invention has industrial applicability as a control variable optimization method, a bioresource production method, and a bioresource production system.

[Reference signs list]

S: Bioresource production system
A: Optimal control variable system
B: Cultivation system
B1: Incubator
B2: Cultivation control device
B3: Various sensors
B4: Sensor control device
C: Control computer

**Claims**

1. A control variable optimization method comprising:

   separating control variables into initial variables and manipulated variables;
   creating a predictive model for said initial variables and a predictive model for said manipulated variables; and
   optimizing said control variables by combining said predictive models.

2. The control variable optimization method according to claim 1, comprising:

   calculating priorities of said initial variables and said manipulated variables from the gradient of the objective variable with respect to said control variables; and

determining an optimization order of said initial variables and said manipulated variables based on the calculated priorities.

3. The control variable optimization method according to claim 1, comprising:
repeatedly performing inverse analysis using said predictive models for said initial variables and said manipulated variables until the optimal control values of said initial variables and said manipulated variables converge, thereby optimizing said control variables.

4. The control variable optimization method according to claim 1, comprising:

assigning medium temperature and pH to said manipulated variables and assigning medium components and an inoculation amount to said initial variables; and
optimizing said control variables by combining said predictive models.

5. The control variable optimization method according to claim 1, comprising:
performing dimensionality reduction on explanatory variables used for said predictive models by extracting coefficients that minimize an approximation error through projection onto a Gompertz curve.

6. The control variable optimization method according to claim 1, comprising:
presenting, upon calculation of said optimal control values of said initial variables and said manipulated variables, a distribution of predicted values of said control variables and a relative position of said optimal control values with respect to said training data.

7. The control variable optimization method according to claim 1, comprising:
separating said manipulated variables into time-indexed variables so that all of said manipulated variables can be treated equivalently to said initial variables.

8. A bioresource production method comprising cultivating or farming an organism based on control variables optimized by said control variable optimization method according to claim 1.

9. A bioresource production system comprising:

a cultivation system for performing bioresource production; and
a control variable optimization system configured to optimize control variables obtained from said cultivation system,
wherein said control variable optimization system is configured to:

separate said control variables into initial variables and manipulated variables;
create a predictive model for said initial variables and a predictive model for said manipulated variables; and
optimize said control variables by combining said predictive models.

FIG.1

FIG.2

Inputs of control variables
for which influence is to be caluculated

|  | Control variable1 |
|---|---|
| Lower limit value | 1 |
| Value within range2 | 2 |
| Value within range3 | 3 |
| Value within range4 | 4 |
| ... |  |
| Upper limit value | 10 |

Prediction

Predictive model

Predictive value
of the objective variable

Visualization of the influence
of control variable on the objective variable

Influence
of the control
variable

Control value

## FIG.3

Multivariate

|  | Target | Medium component1 | Inoculation amount | Temp at 0min | Temp at 10min | ... | Temp at 1000min | SensorX at 0min | SensorX at 10min | ... | SensorX at 1000min |
|---|---|---|---|---|---|---|---|---|---|---|---|
| Batch1 | XXX | XXX | XXX | XXX | XXX | XXX | XXX | XXX | XXX | XXX | XXX |
| Batch2 | XXX | XXX | XXX | XXX | XXX | XXX | XXX | XXX | XXX | XXX | XXX |
| Batch3 | XXX | XXX | XXX | XXX | XXX | XXX | XXX | XXX | XXX | XXX | XXX |

2 variables

|  | Target | Medium component1 | Inoculation amount | Temp at 0min | Temp at 10min | ... | Temp at 1000min | Coefficients a of sensorX | Coefficients b of sensorX |
|---|---|---|---|---|---|---|---|---|---|
| Batch1 | XXX | XXX | XXX | XXX | XXX | XXX | XXX | 10 | 20 |
| Batch2 | XXX | XXX | XXX | XXX | XXX | XXX | XXX | 11 | 25 |
| Batch3 | XXX | XXX | XXX | XXX | XXX | XXX | XXX | 12 | 30 |

Cultivation time
Approximated by $y=a*exp(-b*exp(-c*x))+d$

## FIG.4

FIG.5

FIG.6

(a)  Comparison between measured value
      and approximate value

Gompertz curve
Approximated by y=a*exp(-b*exp(-c*x))+d

Measured values of sensor

The solid glay line: Measured value
The solid black line:Approximate value

Cultivation time

(b) Comparison of prediction accuracy

****

Prediction accuracy(RMSE)

**** : p < 0.0001
Error bar:Standard deviation
RMSE is standardized
by objective variable

Mean of
Without dimensionality reduction
35%
Mean of
With dimensionality reduction

Without
dimensionality reduction

With
dimensionality reduction

FIG.7

EP 4 773 049 A1

FIG.8

FIG.9

Acquired data sets

Real-time converted data

Calculation of optimal control values of initial variables

Real-time Calculation of optimal control values for manipulated variable

Cultivation control device

FIG.10

# EP 4 773 049 A1

## INTERNATIONAL SEARCH REPORT

| | International application No. |
|---|---|
| | **PCT/JP2024/024784** |

| A. | CLASSIFICATION OF SUBJECT MATTER |
|---|---|

*G06N 20/00*(2019.01)i; *C12M 1/00*(2006.01)i; *C12P 1/00*(2006.01)i; *G06N 3/09*(2023.01)i
FI:   G06N20/00 130; C12M1/00 C; C12P1/00; G06N3/09

According to International Patent Classification (IPC) or to both national classification and IPC

| B. | FIELDS SEARCHED |
|---|---|

Minimum documentation searched (classification system followed by classification symbols)

G06N20/00; C12M1/00; C12P1/00; G06N3/09

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | US 2023/0064718 A1 (X DEVELOPMENT LLC) 02 March 2023 (2023-03-02) paragraphs [0012], [0097]-[0139], fig. 5-6 | 1-9 |
| A | WO 2023/286864 A1 (CHITOSE LAB CORP.) 19 January 2023 (2023-01-19) paragraph [0059] | 1-9 |
| A | 庄野 暢晃 他, コンボリューショナルデータを活用したバイオ生産マネジメント. 生物工学, 2020, vol. 98, no. 7, pp. 392-394, [online], [retrieved on 15 August 2024], Internet: <URL: https://www.sbj.or.jp/wp-content/uploads/file/sbj/9807/9807_branch_spirit.pdf>, (SHONO, Nobuaki et al., Bioproduction Management using convolutional data, Seibutsu-kogaku Kaishi) entire text, all drawings | 1-6 |

☐ Further documents are listed in the continuation of Box C.    ☑ See patent family annex.

| | |
|---|---|
| *    Special categories of cited documents: | "T"  later document published after the international filing date or priority date and not in conflict with the application but cited to understand the principle or theory underlying the invention |
| "A"  document defining the general state of the art which is not considered to be of particular relevance | |
| "D"  document cited by the applicant in the international application | "X"  document of particular relevance; the claimed invention cannot be considered novel or cannot be considered to involve an inventive step when the document is taken alone |
| "E"  earlier application or patent but published on or after the international filing date | |
| "L"  document which may throw doubts on priority claim(s) or which is cited to establish the publication date of another citation or other special reason (as specified) | "Y"  document of particular relevance; the claimed invention cannot be considered to involve an inventive step when the document is combined with one or more other such documents, such combination being obvious to a person skilled in the art |
| "O"  document referring to an oral disclosure, use, exhibition or other means | "&"  document member of the same patent family |
| "P"  document published prior to the international filing date but later than the priority date claimed | |

| Date of the actual completion of the international search | Date of mailing of the international search report |
|---|---|
| **15 August 2024** | **27 August 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT
### Information on patent family members

International application No.

**PCT/JP2024/024784**

| Patent document cited in search report | Publication date (day/month/year) | Patent family member(s) | Publication date (day/month/year) |
|---|---|---|---|
| US 2023/0064718 A1 | 02 March 2023 | WO 2023/033885 A1 | |
| WO 2023/286864 A1 | 19 January 2023 | EP 4372371 A1 paragraphs [0111]-[0112] | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **T. HOGIRI et al.** *J. Biosci. Bioeng*, 2018, vol. 125 (2)
- **I. MUTANDA et al.** *Microbiol. Res.*, 2023, 274
- **H. KANEKO et al.** *Anal. Sci. Adv.*, 2021, vol. 2, 470-479